# EUROPEAN PATENT SPECIFICATION

(11) **EP 4 134 101 B1**
(45) Date of publication and mention of the grant of the patent: **04.11.2026**
(21) Application number: 22191949.1
(22) Date of filing: 07.07.2020
(51) Int. Cl.: A61K 31/4985, A61K 31/19, A61K 45/06, A61K 33/00, A61P 25/18

(54) **DEUTERATED LUMATEPERONE FOR THE TREATMENT OF THE BIPOLAR II DISORDER**
DEUTERIERTES LUMATEPERON ZUR BEHANDLUNG DER BIPOLAR-II-STÖRUNG
LUMATEPERONE DEUTEREE POUR LE TRAITEMENT DU TROUBLE BIPOLAIRE II

(30) Priority: 07.07.2019 US 201962871170 P
(43) Date of publication of application: 15.02.2023
(62) Document number(s) of the earlier application(s) in accordance with Art. 76 EPC: 20837066.8 / 3 993 798
(73) Proprietor: Intra-Cellular Therapies, Inc., Titusville, NJ 08560 (US)
(72) Inventor: MATES, Sharon, New York, 10016 (US); DAVIS, Robert, New York, 10016 (US); VANOVER, Kimberly, New York, 10016 (US)
(74) Representative: Carpmaels & Ransford LLP

(56) References cited:
- WO-A1-2017/117514
- ROBERT E. DAVIS ET AL: "ITI-007 in the treatment of schizophrenia: from novel pharmacology to clinical outcomes", EXPERT REVIEW OF NEUROTHERAPEUTICS, vol. 16, no. 6, 2 June 2016 (2016-06-02), GB, pages 601 - 614, XP055404353, ISSN: 1473-7175, DOI: 10.1080/14737175.2016.1174577
- KUMAR B. ET AL: "Lumateperone: a new treatment approach for neuropsychiatric disorders", DRUGS OF TODAY, vol. 54, no. 12, 1 December 2018 (2018-12-01), ES, pages 713, XP093011173, ISSN: 1699-3993, Retrieved from the Internet <URL:http://dx.doi.org/10.1358/dot.2018.54.12.2899443> [retrieved on 20230102], DOI: 10.1358/dot.2018.54.12.2899443
- UNKNOWN: "Corporate Presentation", 30 June 2019 (2019-06-30), XP093011183, Retrieved from the Internet <URL:https://ir.intracellulartherapies.com/static-files/93b08960-f01c-4864-aa22-8cadb3539753> [retrieved on 20230103]

## Description

### TECHNICAL FIELD

The present disclosure relates to use of lumateperone, in tosylate salt form, in deuterated form, for the treatment of Bipolar II Disorder. The invention is defined in the claims.

### BACKGROUND

Substituted heterocycle fused gamma-carbolines such as lumateperone are known to be 5-HT_{2A} or 5-HT_{2A}/D2 receptor ligands, which are useful in treating central nervous system disorders. These compounds antagonize the serotonin-2A (5-HT_{2A}) receptor, and/or modulate dopamine receptor signaling at the level of key intra-cellular phosphoproteins. Such compounds are principally known to be useful for the treatment of positive and negative symptoms of schizophrenia. At dopamine D2 receptors, these compounds have dual properties and act as both post-synaptic antagonists and pre-synaptic partial agonists. They also stimulate phosphorylation of glutamatergic NMDA NR2B, or GluN2B, receptors in a mesolimbic specific manner. It is believed that this regional selectivity in the brain areas thought to mediate the efficacy of antipsychotic drugs, together with the serotonergic, glutamatergic, and dopaminergic interactions, may result in antipsychotic efficacy for positive, negative, affective and cognitive symptoms associated with schizophrenia. The compounds also exhibit serotonin reuptake inhibition, providing antidepressant activity for the treatment of schizoaffective disorder, co-morbid depression, and/or as a stand-alone treatment for major depressive disorder. The 5-HT_{2A} or 5-HT_{2A}/D2 receptor ligands as described are also useful for the treatment of bipolar disorder and other psychiatric and neurodegenerative disorders, particularly behavioral disturbances associated with dementia, autism and other CNS diseases. These features may be able to improve the quality of life of patients with schizophrenia and enhance social function to allow them to more fully integrate into their families and their workplace. These compounds display differential dose-dependent effects, selectively targeting the 5-HT_{2A} receptor at low doses, while progressively interacting with the D2 receptor at higher doses. As a result, at lower doses, they are useful in treating sleep, aggression and agitation. At a high-dose, they can treat acute exacerbated and residual schizophrenia, bipolar disorders, and mood disorders.

Lumateperone, which is 4-((6bR,10aS)-3-methyl-2,3,6b,9,10,10a-hexahydro-lH-pyrido[3',4': 4,5]pyrrolo[1,2,3-de]quinoxalin-8(7H)-yl)-1-(4-fluorophenyl)-1-butanone, having the formula: 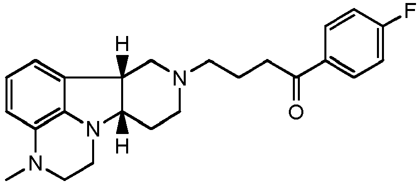 is a therapeutic agent now in clinical trials. Lumateperone provides selective and simultaneous modulation of serotonin, dopamine and glutamate neurotransmission. It exhibits potent (Ki=0.5nM) 5-HT_{2A} receptor antagonism, activity as a mesolimbic/mesocortical-selective dopamine receptor protein phosphorylation modulator consistent with presynaptic D2 receptor partial agonism and postsynaptic D2 receptor antagonism (Ki=32nM) *in vivo*, high D1 receptor affinity (Ki=52nM), and inhibition of the serotonin transporter (SERT) (Ki=26-62nM, using different assays for SERT activity). Lumateperone was approved by the U.S. Food & Drug Administration for the treatment of schizophrenia in 2019, and it is or has been in Phase III clinical development as a treatment for bipolar depression and agitation in dementia, including Alzheimer's Disease.

Lumateperone and related compounds have been disclosed in U.S. Pat. No. 6,548,493; 7,238,690; 6,552,017; 6,713,471; U.S. RE39,680, and U.S. RE39,679, as novel compounds useful for the treatment of disorders associated with 5-HT_{2A} receptor modulation such as anxiety, depression, psychosis, schizophrenia, sleep disorders, sexual disorders, migraine, conditions associated with cephalic pain, and social phobias. PCT/US08/03340 and U.S. Pat. No. 7,081,455 also disclose methods of making substituted heterocycle fused gamma-carbolines and uses of these gamma-carbolines as serotonin agonists and antagonists useful for the control and prevention of central nervous system disorders such as addictive behavior and sleep disorders. WO 2009/145900 and U.S. 8,598,119, and WO 2013/155506 and US 2015/0080404, disclose the use of specific substituted heterocycle fused gamma-carbolines for the treatment of a combination of psychosis and depressive disorders as well as sleep, depressive and/or mood disorders in patients with psychosis or Parkinson's disease and for the treatment or prophylaxis of disorders associated with dementia, particularly behavioral or mood disturbances such as agitation, irritation, aggressive/assaultive behavior, anger, physical or emotional outbursts and psychosis and sleep disorders associated with dementia. WO 2009/114181 and U.S. 8,648,077, and US2020/0157100, disclose methods of preparing toluenesulfonic acid addition salt crystals of particular substituted heterocycle fused gamma-carbolines, e.g., toluenesulfonic acid addition salt of lumateperone (mono-tosylate and bis-tosylate salts).

WO 2011/133224 and U.S. 8,993,572, disclose prodrugs/metabolites of substituted heterocycle fused gamma-carbolines for improved formulation, e.g., extended/controlled release formulation. This application discloses that heterocycle fused gamma-carboline N-substituted with a 4-fluorophenyl(4-hydroxy)butyl moiety are shown to have high selectivity for the serotonin transporter (SERT) relative to the heterocycle fused gamma-carboline containing 4-fluorophenylbutanone.

WO 2009/145900 (and U.S. 8,598,119) teaches that selected substituted heterocycle fused gamma-carboline compounds have nanomolar affinity for the serotonin reuptake transporter (SERT) and so are selective serotonin reuptake inhibitors.

As disclosed in WO2015/154025, US 2017/0183350, WO 2017/165843, and US 2019/231780, deuterated forms of lumateperone and related compounds have been shown to have improved metabolic stability.

According to the National Institute of Mental Health, bipolar disorder, also known as manic-depressive illness, is a brain disorder that causes unusual shifts in mood, energy, activity levels, and the ability to carry out day-to-day tasks. There are four basic types of bipolar disorder; all of them involve clear changes in mood, energy, and activity levels. These moods range from periods of extremely "up," elated, and energized behavior (known as manic episodes) to very sad, "down," or hopeless periods (known as depressive episodes). Less severe manic periods are known as hypomanic episodes.
a. Bipolar I Disorder- defined by manic episodes that last at least 7 days, or by manic symptoms that are so severe that the person needs immediate hospital care. Usually, depressive episodes occur as well, typically lasting at least 2 weeks. Episodes of depression with mixed features (having depression and manic symptoms at the same time) are also possible.
b. Bipolar II Disorder- defined by a pattern of depressive episodes and hypomanic episodes, but not the full-blown manic episodes described above.
c. Cyclothymic Disorder (also called cyclothymia)- defined by numerous periods of hypomanic symptoms as well numerous periods of depressive symptoms lasting for at least 2 years (1 year in children and adolescents). However, the symptoms do not meet the diagnostic requirements for a hypomanic episode and a depressive episode.
d. Other Specified and Unspecified Bipolar and Related Disorders- defined by bipolar disorder symptoms that do not match the three categories listed above.

The etiology of bipolar disorder remains unclear, and the disorder remains resistant to treatment. Pharmaceutical treatments include mood stabilizing drugs, such as lithium or anticonvulsants (e.g., carbamazepine, lamotrigine, and valproate). Conventional antidepressants, such as selective serotonin reuptake inhibitors (SSRIs) are not only less effective for treating bipolar depression than for major depressive disorder, but they may do harm, by triggering manic episodes and rapid cycling.

Antipsychotic medications are generally effective for short-term treatment of bipolar manic episodes in Bipolar I Disorder, but they are generally ineffective in treating Bipolar II Disorder and in maintenance treatment during depressive episodes. Moreover, many antipsychotic medications exhibit significant side effects, such as extrapyramidal symptoms including acute dyskinesias and dystonic reactions, tardive dyskinesia, Parkinsonism, akinesia, akathisia, and neuroleptic malignant syndrome.

For example, the atypical antipsychotic aripiprazole was extensively evaluated for treatment of bipolar disorder, but a review of the clinical data concluded: "Although aripiprazole has proven efficacy for acute mania and the prevention of mania, the evidence available thus far does not support the efficacy of aripiprazole for the treatment of acute bipolar depression and prevention of depressive relapse." Yatham LN, "A clinical review of aripiprazole in bipolar depression and maintenance therapy of bipolar disorder." J. Affect. Disord. 2011 Jan;128 Suppl 1:S21-8. Aripiprazole is currently approved in the United States for "Acute Treatment of Manic and Mixed Episodes associated with Bipolar I." It is not currently approved to treat Bipolar II Disorder.

While there are many antipsychotic agents approved to treat mania associated with Bipolar I Disorder, only four antipsychotic agents are currently approved in the United States to treat depression associated with Bipolar I Disorder, and only one antipsychotic agent is currently approved in the United States to treat Bipolar II Disorder, quetiapine (Seroquel^{®}). Quetiapine, however, has been reported to be highly sedating and to cause a number of metabolic side effects such as hyperglycemia, dyslipidemia, and weight gain. It also has been associated with emergence of suicidal thoughts in some patients.

Estimates vary, but it is believed that approximately 30-60% of patients suffering from bipolar disorders suffer from Bipolar II Disorder. Despite its prevalence, Bipolar II Disorder is often misdiagnosed as either Major Depressive Disorder or Bipolar I Disorder, and improperly treated as a result. There is a need for safer and more effective treatments for Bipolar II Disorder, as well as a need for therapies effective to treat both Bipolar I Disorder and Bipolar II Disorder.

WO 2017/117514 A1 discloses novel 2,3,6b,7,8,9,10,10a-octahydro-1H-pyrido[3',4':4,5]pyrrolo[1,2,3-de]quinoxalines, pharmaceutically acceptable salts thereof and compositions comprising a compound of this invention and the use of such compositions in methods of treating diseases and conditions that are beneficially treated by administering a serotonergic, dopaminergic, and glutamatergic modulator. R. E. Davis et al, Expert Rev. Neurother., 2016, 16, 601-614 discloses IT-007 in the treatment of schizophrenia, from novel pharmacology to clinical outcome. B. Kumar, Drug Today, 2018, 713, discloses lumateperone as a new treatment approach for neuropsychiatric disorders.

### BRIEF SUMMARY

The present invention provides a compound selected from: 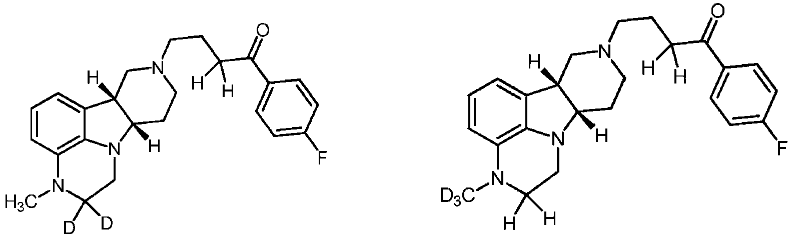 and 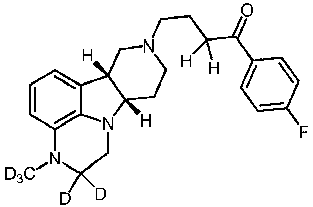 wherein D represents a hydrogen position with substantially greater than natural deuterium incorporation, in tosylate salt form, for use in a method for the treatment of Bipolar II Disorder; wherein the method comprises once daily administration of a tablet or capsule comprising about 60 mg of the compound in tosylate salt form in combination or association with a pharmaceutically acceptable diluent or carrier.

We have surprisingly found that lumateperone is useful to treat Bipolar II Disorder. Clinical results suggest that lumateperone is at least as effective in this indication as existing antipsychotic agents in treating bipolar disorders, with particularly unexpected efficacy in Bipolar II Disorder, which is often resistant to treatment with antipsychotic agents. Moreover, lumateperone exhibits a favorable safety profile. The clinical trials demonstrate that, unlike many other antipsychotic agents, lumateperone does not increase akathisia, restlessness, or other movement disorders, it does not increase suicidal ideation, and it does not have significant metabolic side effects.

Further embodiments will be apparent from the following detailed description and examples.

### DETAILED DESCRIPTION

Any reference in the description to a method of treatment of the human (or animal) body by therapy (or for diagnosis), to the use in a treatment, or to a therapy is to be interpreted as reference to compounds, pharmaceutical compositions or combinations of the present invention for use in such a method, treatment or therapy.

In a Phase 3 clinical trial evaluating lumateperone as monotherapy in the treatment of major depressive episodes associated with Bipolar I or Bipolar II disorder, lumateperone 42 mg daily (administered orally as 60 mg of the tosylate salt) met the primary endpoint for improvement in depression as measured by change from baseline versus placebo on the Montgomery-Åsberg Depression Rating Scale (MADRS) total score (p<0.001; effect size = 0.56), as well as its key secondary endpoint, Clinical Global Impression Scale for Bipolar for Severity of Illness (CGI-BP-S) Total Score (p<0.001; effect size = 0.46).

Moreover, the patients did not exhibit emergence of mania as measured by the Young Mania Rating Scale (YMRS) and a specific clinical global impression of severity for mania, meaning that the drug was effective in preventing the emergence of mania or hypomania, and did not trigger manic episodes or cause rapid cycling that is often linked to SSRIs in treatment of bipolar disorders.

Unexpectedly, in view of the generally limited efficacy of antipsychotics in Bipolar II Disorder, a subgroup analysis of patients with Bipolar II disorder showed that lumateperone at 42 mg daily was statistically significant superior versus placebo on the MADRS total score.

Lumateperone demonstrated a favorable safety profile in this trial, comparable to placebo. Unlike quetiapine, lumateperone did not appear to have any noticeable metabolic effects, such as such as hyperglycemia, dyslipidemia, or weight gain. There were no adverse event reports of suicidal ideation, no suicides, and no discontinuations due to suicidal thoughts. Equally important, there were also no adverse event reports of extrapyramidal symptoms, such as akathisia, restlessness, or other motor side effects.

Akathisia has been associated with the use of most antipsychotic and antidepressant drugs, particularly in bipolar disorders. Akathisia has been linked to suicide. This highlights the clinical importance of lumateperone, as it can treat Bipolar II Disorder without causing akathisia and is therefore less likely to cause suicidal behaviors.

The present invention provides a compound selected from: 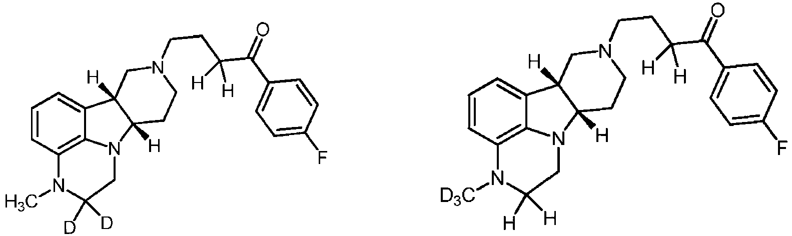 and 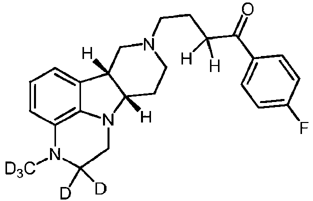 wherein D represents a hydrogen position with substantially greater than natural deuterium incorporation, in tosylate salt form, for use in a method for the treatment of Bipolar II Disorder; wherein the method comprises once daily administration of a tablet or capsule comprising about 60 mg of the compound in tosylate salt form in combination or association with a pharmaceutically acceptable diluent or carrier.

The deuterated form of lumateperone is in the form of the tosylate salt (e.g., an acid addition salt of toluenesulfonic acid, such as a mono-tosylate salt or bis-tosylate salt), optionally wherein the salt is a solid crystalline salt, such as those disclosed in US 2011/112105 and/or US 2020/0157100.

In some embodiments, the deuterium: protium ratio for a specified carbon-bound hydrogen atom is significantly higher, e.g., at least 2x, for example at least 10x higher, than the natural isotope ratios

In some embodiments, D represents a hydrogen position with substantially greater than natural deuterium incorporation (i.e., substantially greater than 0.0156%), e.g., greater than 60%, or greater than 70%, or greater than 80%, or greater than 90% or greater than 95%, or greater than 96%, or greater than 97%, or greater than 98%, or greater than 99%.

In some embodiments, the method comprises once daily administration of a unit dosage for transmucosal administration, e.g., a sublingual or buccal orally disintegrating tablet or film.

In some embodiments, the patient has been diagnosed with Bipolar II Disorder.

In some embodiments, the condition to be treated is depression in Bipolar II Disorder, or a depressive episode associated with Bipolar II Disorder (e.g., a major depressive episode).

In some embodiments, the condition to be treated includes mixed affective episodes in Bipolar II Disorder.

In some embodiments, the condition to be treated includes hypomanic episodes.

In some embodiments, the treatment suppresses mania or hypomania.

In some embodiments, the treatment does not trigger manic or hypomanic episodes or rapid cycling.

In some embodiments, during the course of treatment, the patient does not exhibit emergence of mania or hypomania as measured by the Young Mania Rating Scale (YMRS).

In some embodiments, during the course of treatment, the patient shows improvement in depressive symptoms, e.g., as measured using one or more of the Montgomery-Åsberg Depression Rating Scale (MADRS), the Hamilton Depression Rating Scale (HAM-D), the Beck Depression Inventory (BDI), the Zung Self-Rating Depression Scale, the Wechsler Depression Rating Scale, the Raskin Depression Rating Scale, the Inventory of Depressive Symptomatology (IDS), or the Quick Inventory of Depressive Symptomatology (QIDS)

In some embodiments, during the course of treatment, the patient shows improvement in the Montgomery-Åsberg Depression Rating Scale (MADRS) total score (e.g., improvement within 8 days or less after initiation of treatment).

In some embodiments, following treatment, the patient shows improvement in Clinical Global Impression Scale for Bipolar for Severity of Illness (CGI-BP-S) (e.g., improvement within 8 days or less after initiation of treatment).

In some embodiments, during the course of treatment, the patient shows improvement in Clinical Global Impression Scale for Bipolar Depression for Severity of Illness (CGI- BP- Depression-S) (e.g., improvement within 8 days or less after initiation of treatment).

In some embodiments, during the course of treatment, the patient does not show an increase in Clinical Global Impression Scale for Bipolar Mania for Severity of Illness (CGI-BP-Mania-S).

In some embodiments, during the course of treatment, the patient shows improvement in overall mental health, e.g., as measured using Clinical Global Impression -Severity (CGI-S) or Clinical Global Impression- Improvement (CGI-I) (e.g., improvement within 8 days or less after initiation of treatment).

In some embodiments, the treatment does not result in metabolic side effects, e.g., does not result in one or more of hyperglycemia, dyslipidemia, or weight gain.

In some embodiments, the treatment does not induce suicidal ideation or suicidal thoughts.

In some embodiments, the treatment does not induce extrapyramidal symptoms, e.g., one or more of akathisia, akinesia, restlessness, acute dyskinesia, dystonic reactions, tardive dyskinesia, Parkinson's-like symptoms, or neuroleptic malignant syndrome.

In some embodiments, the treatment does not induce akathisia.

In some embodiments, the deuterated form of lumateperone in tosylate salt form is administered as monotherapy for treatment of Bipolar II Disorder.

In some embodiments, the patient is concurrently receiving a mood-stabilizing agent, e.g., selected from lithium, carbamazepine, oxcarbazepine, lamotrigine, and valproate (including divalproex (a.k.a. valproate semisodium), sodium valproate, and other free, salt or complexed forms of valproic acid).

In some embodiments, the patient is concurrently receiving lithium or valproate.

In some embodiments, the patient is under the age of 18.

In some embodiments, the patient is an adult, e.g., over the age of 18, e.g., between the ages of 18 and 75, inclusive.

In some embodiments, the patient is elderly, e.g., 65 or older, e.g. 75 or older.

In some embodiments, the deuterated form of lumateperone in tosylate salt form is indicated for use in the treatment of depression in Bipolar I Disorder and Bipolar II Disorder.

In some embodiments, the duration of treatment is six weeks or less.

In some embodiments, the treatment is a maintenance treatment.

In some embodiments, the duration of treatment is six weeks or more.

In some embodiments, the patient was previously treated with an SSRI (e.g., such treatment was ineffective and/or discontinued, such as due to side effects).

In some further embodiments, said SSRI is selected from citalopram, escitalopram, fluoxetine, fluvoxamine, paroxetine, and sertraline.

In some embodiments, the patient was previously treated with an antipsychotic, such as an atypical antipsychotic (e.g., such treatment was ineffective and/or discontinued, such as due to side effects).

In some further embodiments, said antipsychotic is selected from haloperidol, aripiprazole, quetiapine, olanzapine, risperidone, lurasidone, paliperidone, iloperidone, ziprasidone, brexipiprazole, asenapine, clozapine, and zotepine.

The words "treatment" and "treating" are to be understood accordingly as embracing prophylaxis and treatment or amelioration of symptoms of disease and/or treatment of the cause of the disease. In particular embodiments, the words "treatment" and "treating" refer to prophylaxis or amelioration of symptoms of the disease.

"Patient" as used herein means a human patient, unless otherwise indicated.

Pharmaceutically acceptable salts of lumateperone include pharmaceutically acceptable acid addition salts, for example, toluenesulfonic acid addition salts (tosylate salts). Tosylate salts of lumateperone include the monotosylate salt and the bis-tosylate salt. Unless otherwise indicated, the term "lumateperone tosylate" refers to the mono-tosylate salt. Lumateperone tosylate is sold as Caplyta^{™}. Where dosages or amounts of a salt are given by weight, e.g., milligrams per day or milligrams per unit dose, the dosage amount of the salt is given as the weight of the corresponding free base, unless otherwise indicated.

The term "concurrently" when referring to a therapeutic use refers to administration of two or more active ingredients to a patient as part of a regimen for the treatment of a disease or disorder, whether the two or more active agents are given at the same or different times or whether given by the same or different routes of administrations. Concurrent administration of the two or more active ingredients may be at different times on the same day, or on different dates or at different frequencies.

The present invention comprises once daily administration of a tablet or capsule comprising about 60 mg of the deuterated form of lumateperone in tosylate salt form in combination or association with a pharmaceutically acceptable diluent or carrier. Dosages employed in practicing the present disclosure will of course vary depending, e.g. on the particular disease or condition to be treated, the particular active compounds used, the mode of administration, and the therapy desired. The deuterated form of lumateperone in tosylate salt form may be administered by any suitable route, including oral, parenteral, transdermal, or transmucosal, for example in the form of a tablet, a capsule, a subcutaneous injection, long acting injectable (depot), or an oral, rapidly disintegrating tablet or film for sublingual or buccal administration.

The deuterated form of lumateperone in tosylate salt form is provided as a tablet or capsule for oral administration, comprising the deuterated form of lumateperone in tosylate salt form in combination with a pharmaceutically acceptable diluent or carrier.

In some embodiments, the deuterated form of lumateperone in tosylate salt form is provided as a rapidly disintegrating tablet for sublingual or buccal administration, comprising the deuterated form of lumateperone in tosylate salt form in combination with a pharmaceutically acceptable diluent or carrier.

For the avoidance of doubt, any disclosure of a numerical range, e.g., "up to X" amount is intended to include the upper numerical limit X. Therefore, a disclosure of "up to 60 mg" is intended to include 60 mg.

In the event of a conflict in a definition in the present disclosure and that of a cited reference, the present disclosure controls.

The disclosure is further illustrated in the following examples, which are meant to be exemplary and not limiting.

### Example 1: Clinical Trial in Bipolar I Disorder and Bipolar II Disorder patients

A multi-center, multi-national, randomized, double-blind, fixed-dose, placebo-controlled clinical trial is conducted substantially in accordance with the following protocol.

Patients are recruited according to the following criteria:
- Major Inclusion Criteria: Male or female subjects of any race, ages 18-75 inclusive, with a clinical diagnosis of Bipolar I or Bipolar II disorder, experiencing a current major depressive episode, and able to provide written informed consent.
- Major Exclusion Criteria: Any female subject who is pregnant or breast-feeding, and any subject judged to be medically inappropriate for study participation.

A total of 381 patients recruited in accordance with the above criteria are randomized 1: 1 to two study arms: (i) lumateperone 42 mg (administered orally as 60 mg of lumateperone tosylate) and (ii) placebo. In the lumateperone arm, lumateperone (lumateperone tosylate 60 mg) is administered once daily every evening for 6 weeks. In the placebo arm, placebo is administered once daily every evening for 6 weeks. The patients do not receive other medications for treatment of bipolar disorders. The study is quadruple-masked (i.e., to participant, care provider, investigator, and outcomes assessor).

The total study duration is about 10 weeks, including up to 2 weeks screening period (washout of prohibited medications), a 6-week double-blind treatment period, and 2-week safety follow-up period.

The primary outcome measure is the Montgomery-Åsberg Depression Rating Scale (MADRS) [Time Frame: Day 43]. Secondary outcome measures are the Clinical Global Impression Scale, Bipolar version (CGI-BP) [Time Frame: Day 43] and the Quality of Life Enjoyment and Satisfaction Questionnaire - Short Form (Q-LES-Q-SF) [Time Frame: Day 43].

A further objective of the study is to determine the safety and tolerability of lumateperone versus placebo as measured by:
a. Incidence of Adverse Events (AEs)
b. Young Mania Rating Scale (YMRS)
c. Columbia Suicide Severity Rating Scale (C-SSRS)
d. Abnormal Involuntary Movement Scale (AIMS)
e. Barnes Akathisia Rating Scale (BARS)
f. Simpson Angus Scale (SAS)
g. Clinical laboratory evaluations
h. Electrocardiograms (ECGs)
i. Vital sign measurements
j. Physical examination and neurological findings

The patient disposition is as follows:

**Table 1**

| | Lumateperone 42 mg | Placebo | Total |
|---|---|---|---|
| Screened | | | 546 |
| Randomized | 191 | 190 | 381 |
| Safety Population | 188 | 189 | 377 |
| Intention to Treat (ITT) Population | 188 | 188 | 376 |
| | | | |
| Completed, n (%) | 167 (87.4) | 166 (87.4) | 333 (87.4) |
| Discontinued, n (%) | 21 (11.0) | 23 (12.1) | 44 (11.5) |

The change seen from baseline to Day 43 in MADRS total score was as follows:

**Table 2**

| Treatment Group | n | Baseline Mean (SD) | n | LS Mean (SE) Change at Day 43 | 95% CI | Comparison to Placebo at Day 43 | | | |
|---|---|---|---|---|---|---|---|---|---|
| | | | | | | LSMD (SE) | 95% CI | Effect Size | P-Value |
| Lumateperone 42 mg N=188 | 169 | 30.7 (4.74) | 169 | -16.7 (0.69) | (-18.06, -15.34) | 4.6 (0.89) | (-6.34, -2.83) | -0.56 | <.001 |
| Placebo N=188 | 166 | 30.2 (4.68) | 166 | -12.1 (0.68) | (-13.45, -10.79) | - | - | - | - |

The change seen from baseline to Day 43 in CGI-BP-S total score was as follows:

**Table 3**

| Treatment Group | n | Baseline Mean (SD) | n | LS Mean (SE) Change at Day 43 | 95% CI | Comparison to Placebo at Day 43 | | | |
|---|---|---|---|---|---|---|---|---|---|
| | | | | | | LSMD (SE) | 95% CI | Effect Size | P-Value |
| Lumateperone 42 mg N=188 | 169 | 10.3 (1.10) | 169 | -3.5 (0.17) | (-3.81, -3.14) | 0.9 (0.22) | (-1.37, -0.51) | -0.46 | <.001 |
| Placebo N=188 | 166 | 10.2 (1.07) | 166 | -2.5 (0.17) | (-2.86, -2.21) | - | - | - | - |

Lumateperone 42 mg was thus superior to placebo as demonstrated by statistically significant improvements on MADRS Total Score and CGI-BP-S which were the primary and key secondary measures in this study. The improvements seen in this study with Lumateperone 42 mg are considered to be clinically meaningful.

The safety profile of lumateperone was similar to placebo. A summary of treatment-emergent adverse events (TEAEs) is as follows:

**Table 4**

| | | Lumateperone 42 mg (N=188) | Placebo (N=189) |
|---|---|---|---|
| | | n (%) | n (%) |
| Number of Patients | | | |
| | With One or More TEAE | 103 (54.8) | 95 (50.3) |
| | With Drug-related TEAE | 78 (41.5) | 59 (31.2) |
| | With Treatment-Emergent SAE | 1 (0.5) | 0 |
| With Drug-related Treatment-Emergent SAE | | 0 | 0 |
| | With Treatment-Emergent Death | 0 | 0 |
| Subjects Discontinued Study | | | |
| | Due to TEAE | 11 (5.9) | 4 (2.1) |
| | Due to Drug-related TEAE | 9 (4.8) | 2 (1.1) |
| | Due to Treatment-Emergent SAE | 1 (0.5) | 0 |
| Due to Drug-related Treatment-Emergent SAE | | 0 | 0 |
| Subjects who Died | | 0 | 0 |

Lumateperone 42 mg was generally safe and well tolerated. The most commonly reported adverse events that were observed at a rate greater than 5% and higher than placebo were headache, somnolence and nausea. Importantly, the rates of akathisia and extrapyramidal symptoms were less than 1% and similar to placebo.

In this trial, once-daily lumateperone 42 mg met the primary endpoint with statistically significant greater improvement over placebo at week 6 (trial endpoint), as measured by change from baseline on the MADRS total score. In the intent-to-treat (ITT) study population, the least squares (LS) mean reduction from baseline for lumateperone 42 mg was 16.7 points, versus 12.1 points for placebo (LS mean difference = 4.6 points; effect size = 0.56, p<0.001). Moreover, lumateperone 42 mg showed statistically significant separation from placebo as early as week 1, which was maintained at every time point throughout the entire trial.

Lumateperone 42 mg also met the key secondary endpoint of statistically significant improvement on the CGI-BP-S Total Score (p<0.001; effect size = 0.46) and on the CGI component that specifically assesses depression (CGI- S Depression Score; p<0.001; effect size = 0.5).

These results were supported by statistically significant benefits on responder rates and remission rates, demonstrating the clinical meaningfulness of the primary outcome. In addition, in a subgroup analysis of patients with Bipolar II disorder lumateperone 42 mg was statistically significant superior to placebo on the MADRS total score.

## Claims

1. A compound selected from: 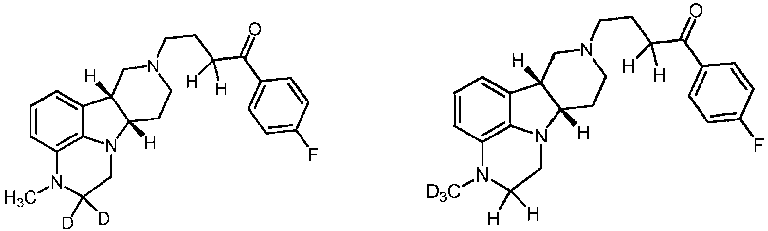 and 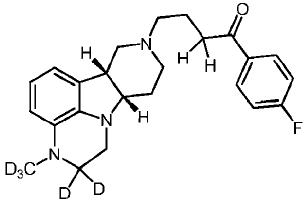 wherein D represents a hydrogen position with substantially greater than natural deuterium incorporation, in tosylate salt form, for use in a method for the treatment of Bipolar II Disorder; wherein the method comprises once daily administration of a tablet or capsule comprising about 60 mg of the compound in tosylate salt form in combination or association with a pharmaceutically acceptable diluent or carrier.

2. The compound for the use of claim 1, wherein the compound is:

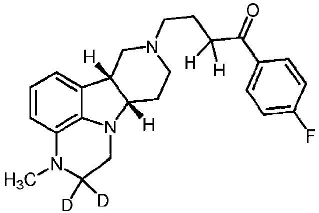

3. The compound for the use of any foregoing claim, wherein the condition to be treated is depression in Bipolar II Disorder, or a depressive episode associated with Bipolar II Disorder (e.g., a major depressive episode).

4. The compound for the use of any foregoing claim, wherein the compound is administered to provide improvement in the Montgomery-Åsberg Depression Rating Scale (MADRS) total score and/or improvement in Clinical Global Impression Scale for Bipolar for Severity of Illness within 8 days or less after initiation of treatment.

5. The compound for the use of any foregoing claim, wherein the compound is administered without causing one or more of hyperglycemia, dyslipidemia, weight gain, suicidal ideation or suicidal thoughts, or extrapyramidal symptoms including akathisia.

6. The compound for the use of any foregoing claim, wherein the patient was previously treated with an SSRI (e.g., such treatment was ineffective and/or discontinued, such as due to side effects).

7. The compound for the use of any foregoing claim, wherein the patient was previously treated with an antipsychotic, such as an atypical antipsychotic (e.g., such treatment was ineffective and/or discontinued, such as due to side effects).

8. The compound for the use of claim 7, wherein said antipsychotic is selected from haloperidol, aripiprazole, quetiapine, olanzapine, risperidone, lurasidone, paliperidone, iloperidone, ziprasidone, brexipiprazole, asenapine, clozapine, and zotepine.

9. The compound for the use according to any one of claims 1-8, wherein the patient is concurrently receiving lithium, carbamazepine, oxcarbazepine, lamotrigine, valproic acid or a valproate salt or complex.

10. The compound for the use according to claim 9, wherein the patient is concurrently receiving lithium, valproic acid or a valproate salt or complex.

11. The compound for the use according to claim 10, wherein the valproate salt or complex is valproate semisodium or sodium valproate.

12. The compound for the use according to any one of claims 1-11, wherein the duration of treatment is six weeks or more.

## Patentansprüche

1. Verbindung, die aus Folgendem ausgewählt ist: 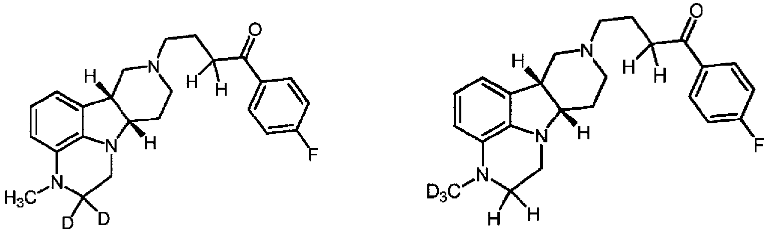 und 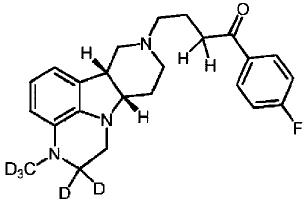 wobei D eine Wasserstoffposition mit im Wesentlichen größerer als natürlicher Deuteriumeinarbeitung, in Tosylatsalzform, zur Verwendung in einem Verfahren für die Behandlung einer Bipolar-II-Störung darstellt; wobei das Verfahren eine einmal tägliche Verabreichung einer Tablette oder Kapsel umfasst, umfassend etwa 60 mg der Verbindung in Tosylatsalzform in Kombination oder Assoziation mit einem pharmazeutisch akzeptablen Verdünnungsmittel oder Träger.

2. Verbindung zur Verwendung nach Anspruch 1, wobei die Verbindung Folgendes ist:

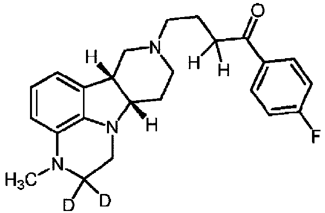

3. Verbindung zur Verwendung nach einem der vorstehenden Ansprüche, wobei der zu behandelnde Zustand Depression bei Bipolar-II-Störung oder eine mit Bipolar-II-Störung assoziierte depressive Episode (z. B. eine schwere depressive Episode) ist.

4. Verbindung zur Verwendung nach einem der vorstehenden Ansprüche, wobei die Verbindung verabreicht wird, um eine Verbesserung in dem Gesamtscore der Montgomery-Åsberg Depression Rating Scale (MADRS) und/oder eine Verbesserung in der Clinical Global Impression Scale for Bipolar for Severity of Illness innerhalb von 8 Tagen oder weniger nach Beginn der Behandlung bereitzustellen.

5. Verbindung zur Verwendung nach einem der vorstehenden Ansprüche, wobei die Verbindung verabreicht wird, ohne eines oder mehrere von Hyperglykämie, Dyslipidämie, Gewichtszunahme, Suizidalität oder Suizidgedanken oder extrapyramidalen Symptomen einschließlich Akathisie zu verursachen.

6. Verbindung zur Verwendung nach einem der vorstehenden Ansprüche, wobei der Patient zuvor mit einem SSRI behandelt wurde (z. B. war eine solche Behandlung unwirksam und/oder wurde abgebrochen, wie aufgrund von Nebenwirkungen).

7. Verbindung zur Verwendung nach einem der vorstehenden Ansprüche, wobei der Patient zuvor mit einem Antipsychotikum behandelt wurde, wie einem atypischen Antipsychotikum (z. B. war eine solche Behandlung unwirksam und/oder wurde abgebrochen, wie aufgrund von Nebenwirkungen).

8. Verbindung zur Verwendung nach Anspruch 7, wobei das Antipsychotikum aus Haloperidol, Aripiprazol, Quetiapin, Olanzapin, Risperidon, Lurasidon, Paliperidon, Iloperidon, Ziprasidon, Brexipiprazol, Asenapin, Clozapin und Zotepin ausgewählt ist.

9. Verbindung zur Verwendung nach einem der Ansprüche 1 bis 8, wobei der Patient gleichzeitig Lithium, Carbamazepin, Oxcarbazepin, Lamotrigin, Valproinsäure oder ein Valproatsalz oder einen Valproatkomplex erhält.

10. Verbindung zur Verwendung nach Anspruch 9, wobei der Patient gleichzeitig Lithium, Valproinsäure oder ein Valproatsalz oder einen Valproatkomplex erhält.

11. Verbindung zur Verwendung nach Anspruch 10, wobei das Valproatsalz oder der Valproatkomplex Valproatseminatrium oder Natriumvalproat ist.

12. Verbindung zur Verwendung nach einem der Ansprüche 1 bis 11, wobei die Dauer der Behandlung sechs Wochen oder mehr beträgt.

## Revendications

1. Composé choisi parmi : 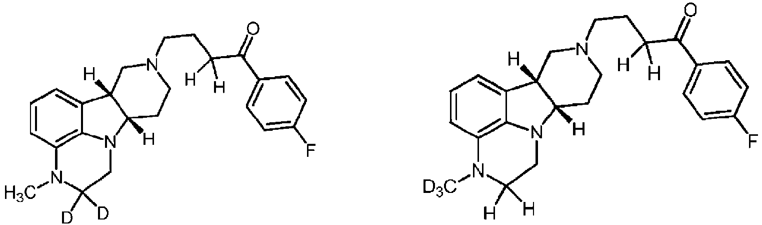 et 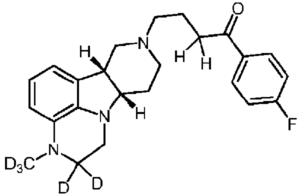 dans lequel D représente une position d'hydrogène avec une incorporation de deutérium sensiblement supérieure à l'incorporation naturelle, sous forme de sel de tosylate, pour une utilisation dans un procédé de traitement du trouble bipolaire de type II ; dans lequel le procédé comprend l'administration une fois par jour d'un comprimé ou d'une capsule comprenant environ 60 mg du composé sous forme de sel de tosylate en combinaison ou en association avec un diluant ou un véhicule pharmaceutiquement acceptable.

2. Composé pour l'utilisation selon la revendication 1, dans lequel le composé est :

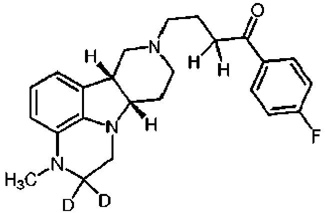

3. Composé pour l'utilisation selon l'une quelconque des revendications précédentes, dans lequel l'affection à traiter est la dépression dans le trouble bipolaire de type II, ou un épisode dépressif associé au trouble bipolaire de type II (par exemple, un épisode dépressif majeur).

4. Composé pour l'utilisation selon l'une quelconque des revendications précédentes, dans lequel le composé est administré pour procurer une amélioration du score total à l'échelle d'évaluation de la dépression de Montgomery-Åsberg (MADRS) et/ou une amélioration à l'échelle d'impression clinique globale pour le trouble bipolaire pour la sévérité de la maladie dans un délai de 8 jours ou moins après le début du traitement.

5. Composé pour l'utilisation selon l'une quelconque des revendications précédentes, dans lequel le composé est administré sans provoquer un ou plusieurs parmi hyperglycémie, dyslipidémie, prise de poids, idéation suicidaire ou pensées suicidaires, ou symptômes extrapyramidaux y compris akathisie.

6. Composé pour l'utilisation selon l'une quelconque des revendications précédentes, dans lequel le patient a déjà été traité avec un ISRS (par exemple, un tel traitement a été inefficace et/ou interrompu, tel qu'en raison d'effets secondaires).

7. Composé pour l'utilisation selon l'une quelconque des revendications précédentes, dans lequel le patient a déjà été traité par un antipsychotique, tel qu'un antipsychotique atypique (par exemple, un tel traitement a été inefficace et/ou interrompu, tel qu'en raison d'effets secondaires).

8. Composé pour l'utilisation selon la revendication 7, dans lequel ledit antipsychotique est choisi parmi halopéridol, aripiprazole, quétiapine, olanzapine, rispéridone, lurasidone, palipéridone, ilopéridone, ziprasidone, brexipiprazole, asénapine, clozapine et zotépine.

9. Composé pour l'utilisation selon l'une quelconque des revendications 1 à 8, dans lequel le patient reçoit simultanément du lithium, carbamazépine, oxcarbazépine, lamotrigine, acide valproïque ou un sel ou complexe de valproate.

10. Composé pour l'utilisation selon la revendication 9, dans lequel le patient reçoit simultanément du lithium, acide valproïque ou un sel ou complexe de valproate.

11. Composé pour l'utilisation selon la revendication 10, dans lequel le sel ou complexe de valproate est le valproate semisodique ou le valproate de sodium.

12. Composé pour l'utilisation selon l'une quelconque des revendications 1 à 11, dans lequel la durée du traitement est de six semaines ou plus.
